# EUROPEAN PATENT SPECIFICATION

(11) **EP 4 466 257 B1**
(45) Date of publication and mention of the grant of the patent: **19.08.2026**
(21) Application number: 23701448.5
(22) Date of filing: 20.01.2023
(51) Int. Cl.: C07D 309/12, C08G 63/08

(54) **METHOD FOR PROCESSING LACTIDES**
VERFAHREN ZUR VERARBEITUNG VON LACTIDEN
PROCÉDÉ DE TRAITEMENT DE LACTIDES

(30) Priority: 21.01.2022 EP 22152812
(43) Date of publication of application: 27.11.2024
(73) Proprietor: Purac Biochem B.V., 4206 AC Gorinchem (NL)
(72) Inventor: DE VRIES, Hans, 4206 AC Gorinchem (NL); GOBIUS DU SART, Gerrit, 4206 AC Gorinchem (NL); VAN KRIEKEN, Jan, 4206 AC Gorinchem (NL)
(74) Representative: De Vries & Metman
(86) International application number: PCT/EP2023/051360
(87) International publication number: WO 2023/139207

(56) References cited:
- WO-A1-2010/105142
- WO-A1-2013/186540
- WO-A2-2010/105143
- US-A1- 2016 280 908
- BOTVIN VLADIMIR ET AL: "Syntheses and chemical transformations of glycolide and lactide as monomers for biodegradable polymers", POLYMER DEGRADATION AND STABILITY, BARKING, GB, vol. 183, 12 November 2020 (2020-11-12), XP086475149, ISSN: 0141-3910, [retrieved on 20201112], DOI: 10.1016/J.POLYMDEGRADSTAB.2020.109427
- SEONG-HO KIM ET AL: "Solid-phase synthesis of enantio-controlled lactic acid oligomers", TETRAHEDRON ASYMMETRY, PERGAMON PRESS LTD, OXFORD, GB, vol. 22, no. 14, 24 August 2011 (2011-08-24), pages 1499 - 1504, XP028322728, ISSN: 0957-4166, [retrieved on 20110905], DOI: 10.1016/J.TETASY.2011.08.021

## Description

### Technical field of the invention

The present invention relates to a process for processing a crude lactide stream, to a process for producing a polylactide, and to a process for producing a racemic mixture of (S)-lactic acid and (R)-lactic acid.

### Background and summary of the invention

Polylactide, also known as poly(lactic acid) or PLA, is a polymer that finds application in a variety of fields, ranging from packaging to disposable tableware. Polylactide is a polymer derived from lactic acid. Lactic acid is a chiral molecule and so exists as either (S)- or (R)-lactic acid. Commercially available polylactides generally contain a large proportion of (S)-lactic acid units and are usually obtained by ring-opening polymerization of (predominantly) L-lactide, a dimer of (S)-lactic acid.

Lactides are commonly synthesized by oligomerizing lactic acid to form lactic acid oligomers via a polycondensation reaction. These oligomers are then depolymerized to form a crude lactide comprising L-lactide ((S,S)-lactide), D-lactide ((R,R)-lactide), and meso-lactide ((S,R)-lactide). Reference is made to, for example, US 5,357,035, US 5,521,278, WO 2010/105143, and US 2014/031566. When the oligomers are synthesized from (S)-lactic acid, the resulting oligomer will contain mostly (S)-lactic acid units. Depolymerization of such an oligomer will, in turn, lead to a crude lactide, wherein L-lactide is the predominant stereoisomer. Under certain circumstances, lactides can be converted into different stereoisomers by means of a racemization process.

L-lactide, D-lactide, and meso-lactide can be sent to a polymerization reactor in various ratios. The ratio in which the lactides are sent to the polymerization reactor determines the ratio of (S)-lactic acid units to (R)-lactic acid units in the polymer, as each L-lactide molecule contributes two (S)-lactic acid units to the polymer, each D-lactide molecule contributes two (R)-lactic acid units to the polymer, and each meso-lactide molecule contributes one (S)-lactic acid unit and one (R)-lactic acid unit to the polymer. The ratio of (S)-lactic acid units to (R)-lactic acid units in the polymer is important, because it determines, for a large part, the physical properties of the polylactide. A polylactide comprising mainly (S)-lactic units and, say, 10% of (R)-lactic acid units will, for example, be less crystalline than a polylactide comprising only (S)-lactic acid units.

However, the ratio of (S)-lactic acid units to (R)-lactic acid units in the polymer is not the only factor that contributes to the physical properties of the polylactide. Another important factor is the presence or absence of impurities, such as hydroxyl-containing impurities, in the lactide stream that is sent to the polymerization reactor. Hydroxyl-containing impurities, if present in the lactide stream that is polymerized, reduce the average molecular weight of the resulting polylactide, as described in US 5,357,035. Impurities may also affect the color of the polylactide, its thermal stability and its suitability for food contact applications.

WO 2010/105143 describes a process wherein meso-lactide is separated from a crude lactide and recycled to an oligomerization reactor (for the oligomerization of lactic acid via a polycondensation reaction) and/or a depolymerization reactor. The conditions in these reactors are such that a portion of the recycled meso-lactide is then converted to either L- or D-lactide. The reasons for doing so are twofold. First, the recycling of meso-lactide leads to an increase in the non-predominant lactide (being D-lactide or L-lactide, depending on whether the lactide synthesis started with (S)-lactic acid or (R)-lactic acid), relative to a process wherein no meso-lactide is recycled. This is said to be advantageous, because the (R)-lactic acid units can then be introduced into the polylactide through D-lactide instead of through meso-lactide. Second, the meso-lactide stream obtained in WO 2010/105143 is rich in so-called "intermediate-boiling impurities," which result in discoloration of the polylactide if they are present in the lactide stream that is sent to the polymerization reactor. By recycling the meso-lactide to the oligomerization reactor and/or the depolymerization reactor, the impurities are partly kept away from the polymerization reactor, and an increased amount of D-lactide may be separated and used in a purified L- and D-lactide stream directly for polymerization.

Thus, in essence, the process described in WO 2010/105143 aims to reduce the need to use meso-lactide in the lactide stream sent to the polymerization reactor by recycling and converting meso-lactide into L- and D-lactide. A first disadvantage of this process is that the conversion of meso-lactide into L- and D-lactide is carried out under conditions that also result in the conversion of L- and D-lactide into meso-lactide, which limits the overall efficiency of the process. A second disadvantage of the process may be that the recycling of the meso-lactide stream contaminates the main lactide synthesis reactor, because impurities are concentrated in the meso-lactide stream being recycled. Moreover, any meso-lactide that is sent (directly) from the last distillation step to the polymerization reactor will contain some impurities, which will result in a discolored product being synthesized. This may cause separation by distillation to become ineffective and increases the overall amount of impurities in all distillation fractions A further disadvantage to this process is that it results in the build-up of the less dominant lactide in the process, as this keeps being recycled to the beginning of the process.

In the art, processes in which meso-lactide streams are subjected to racemization have also been described.US 2014/031566 describes a process wherein meso-lactide is separated from a crude lactide stream to form a meso-lactide stream. The meso-lactide is then subjected to racemization, thereby forming L- and D-lactide. Here, too, the conditions for the racemization are such that conversion of L- and D-lactide to meso-lactide occurs. An enriched meso-lactide stream may remain from the racemized mixture and can be handled in various ways. The stream may be discarded or used in low-value applications. It is said the "intermediate-boiling impurities" present in the enriched meso-lactide stream may be removed by extraction or by a chemical treatment method. The chemical treatment method involves converting either the "intermediate-boiling impurities" or the meso-lactide into a different chemical species. It is said this different chemical species can then more easily be separated from the "intermediate-boiling impurities" or the meso-lactide. No mention is made of the species into which the "intermediate-boiling impurities" or the meso-lactide should be converted to facilitate separation.

WO2010105142 is directed to a process for recovering lactide by subjecting a lactide stream to a catalytic racemization step. It also describes the removal of meso-lactide from the rest of the lactide stream to remove the non-dominant lactide and impurities. In in a preferred embodiment this meso-lactide fraction is subjected to the racemization step. Again, the conditions prevailing during racemization are such that conversion of L- and D-lactide to meso-lactide occurs, resulting in a decrease in yield.

There is a need in the art for a process that specifically converts meso-lactide into L-lactide and D-lactide (i.e., without also converting significant portions of L- and D-lactide into meso-lactide), that allows for containment of acid-containing impurities and their facile removal from the process, and that is not associated with build-up of non-dominant lactide. The present invention provides such a process.

In an aspect, the invention relates to a process for processing a crude lactide stream, wherein the process comprises the steps of:
separating a crude lactide stream comprising 75-95 wt.% of L-lactide (based on the total weight of lactide in the stream), 0.01 to 5 wt.% of D-lactide (based on the total weight of lactide in the stream), and 1-25 wt.% of meso-lactide (based on the total weight of lactide in the stream) in one or more steps to form a meso-lactide stream comprising at least 50 wt.% of meso-lactide (based on the total weight of lactide in the stream) and at least one purified lactide stream comprising L-lactide and D-lactide, wherein the amount of meso-lactide in the at least one purified L- and/or D lactide stream is at most 50% of the amount of meso-lactide in the crude lactide stream;
oligomerizing the meso-lactide stream to form an oligomer-containing stream comprising lactic acid oligomers; and
depolymerizing the oligomer-containing stream to form a product stream comprising meso-lactide, L-lactide, and D-lactide.

A key feature of the present invention is that the oligomerization-depolymerization sequence used in the process according to the invention specifically converts a stream containing at least 50 wt.% of meso-lactide (via a lactic acid oligomer) into significant portions of L- and D-lactide. Because the oligomerization takes place in the absence of substantial amounts of L- and D-lactides, generally in a dedicated oligomerization reactor, the - undesired - conversion of these compounds into meso-lactide is limited. This means that energy is efficiently spent on converting a relatively low-value intermediate (meso-lactide) into high-value intermediates (Land D-lactides). Indeed, as shown in the Examples, the conversion of meso-lactide into L- and D-lactides occurs to a high degree. Such a high conversion is not achieved when meso-lactide is recycled and subjected to a racemization process as described in e.g. WO 2010/105143, WO2010/105142US 2014/031566.

Additionally, because in the process of the present invention lactate units are rearranged via an intermediate oligomer, not through racemization (although racemization of specific fractions is not excluded), the yield loss which occurs when meso-lactide is subjected to a racemization step is prevented. Further, in the process of the present invention build-up of non-dominant lactic acid enantiomer is prevented while at the same time high-value intermediates (L- and D-lactides) are generated.

The process according to the invention also allows for the conversion of meso-lactide into Land D-lactide without contamination of the main lactide synthesis reactor with recycled meso-lactide and/or impurities. The meso-lactide and/or the impurities are contained in other parts of the reactor where they do not disturb the main lactide synthesis. This results in a more stable process, meaning that the contents of the main lactide synthesis (such as the contents of the crude lactide stream) are surprisingly consistent over time.

In another aspect, the invention relates to a process for producing a polylactide, wherein the process comprises obtaining at least one stream according to the process for processing a crude lactide according to invention, providing at least a portion of the at least one stream, directly or indirectly, that is with or without intermediate processing,, to a polymerization reactor, and polymerizing the at least one stream to form a polylactide.

In yet another aspect, the invention relates to a process for producing a racemic mixture of lactic acid, wherein the process comprises hydrolyzing at least a portion of the stream comprising a racemic mixture of L-lactide and D-lactide obtained according to the process for processing a crude lactide according to the invention to form a racemic mixture of (S)-lactic acid and (R)-lactic acid.

### Detailed description

The aspects of the invention will be discussed in more detail below. Specific advantages of the processes, as well as of specific embodiments thereof, will become apparent from the further specification.

### Process for processing a crude lactide

As mentioned above, disclosed herein is a process for processing a crude lactide stream, wherein the process comprises the steps of
separating a crude lactide stream comprising 75-95 wt.% of L-lactide (based on the total weight of lactide in the stream), 0.01 to 5 wt.% of D-lactide (based on the total weight of lactide in the stream), and 1-25 wt.% of meso-lactide (based on the total weight of lactide in the stream) in one or more steps to form a meso-lactide stream comprising at least 50 wt.% of meso-lactide (based on the total weight of lactide in the stream) and at least one purified lactide stream comprising L-lactide and/or D-lactide, wherein the amount of meso-lactide in the at least one purified L- and/or D lactide stream is at most 50% of the amount of meso-lactide in the crude lactide stream;
oligomerizing the meso-lactide stream to form an oligomer-containing stream comprising lactic acid oligomers; and
depolymerizing the oligomer-containing stream to form a product stream comprising meso-lactide, L-lactide, and D-lactide.

The crude lactide stream comprises L-lactide, D-lactide, and meso-lactide. The crude lactide comprises from 75 to 95 wt.% of L-lactide (based on the total weight of lactide in the stream), preferably 80 to 95 wt.%. The crude lactide comprises from 0.01 to 5 wt.% of D-lactide (based on the total weight of lactide in the stream). The crude lactide comprises from 1 to 25 wt.% of meso-lactide (based on the total weight of lactide in the stream). The amount of meso-lactide in a composition may be determined by HPLC, e.g. using water/acetonitrile mixtures as eluent and UV-detection; lactic acid and lactoyl lactic acid will elute first, then meso-lactide, then Land D-lactic acid, and then oligomers of lactic acid. The crude lactide stream may comprise residual amounts of lactic acid, lactic esters, and/or water, which can be removed by means of distillation, crystallization, or extraction. These residual amounts, if present, may be removed before or during the separation step, but are preferably removed before the separation step. The crude lactide stream may comprise acid-containing impurities, such as lactoyl lactic acid, succinic acid and acetic acid. The acid-containing impurities may be present in the crude lactide stream in an amount such that the crude lactide stream has a free acid content of at least 20 meq/kg, in particular at least 50 meq/kg, and/or at most 150 meq/kg. As a maximum, the acid-containing impurities may be present in the crude lactide stream in an amount of at most 250 meq/kg. The free acid content as used herein can be determined by means of titration, e.g. using sodium methylate or potassium methylate in water-free methanol.

The crude lactide stream may be separated by any suitable separation technique. It is preferred that the crude lactide stream is separated by means of distillation or crystallization (e.g. solvent crystallization or melt crystallization) or any combination of both. These separation techniques will separate the meso-lactide and at least a portion of the acid-containing impurities, if present, from the L- and D-lactide. This results in the formation of a meso-lactide stream comprising meso-lactide (and optionally acid-containing impurities) and at least one purified lactide stream comprising L-lactide and/or D-lactide. In a preferred process, at least one distillation step is used to separate the crude lactide stream. If a distillation is used, other streams taken from the distillation may be treated as purge streams, such as an overhead stream containing volatile components (e.g., water, lactic, formic and acetic acid).

The at least one purified lactide stream comprising D-lactide and/or L-lactide are relatively pure, when compared to the crude lactide. Specifically, the amount of meso-lactide and the amount of (volatile) acid-containing impurities in the at least one purified lactide stream are lower than in the crude lactide stream. The amount of meso-lactide in the at least one purified D- and/or L-lactide stream is at most 50% of the amount of meso-lactide in the crude lactide stream, preferably at most 25%, more preferably at most 10%.

It is preferred that the at least one purified lactide stream comprises predominantly L-lactide. Therefore, the purified lactide stream(s) may comprise at least 80 wt.% of L-lactide (based on the total weight of lactide in the stream), preferably at least 85 wt.% of L-lactide, more preferably at least 90 wt.% of L-lactide, even more preferably at least 95 wt.% of L-lactide. In more preferred embodiments, at least one of the purified lactide streams comprises an amount of L-lactide as defined above (i.e., at least 80 wt.%, or a preferred amount of L-lactide as specified above, based on the total weight of lactide in the stream) and 5 wt.% or less of D-lactide, preferably 3 wt.% or less of D-lactide, more preferably 1 wt.% or less of D-lactide. In still more preferred embodiments, at least one of the purified lactide streams comprises 5 wt.% or less of meso-lactide (based on the total weight of lactide in the stream), preferably 4 wt.% or less of meso-lactide, more preferably 3 wt.% or less of meso-lactide, even more preferably 2 wt.% or less of meso-lactide (preferably, in combination with a preferred amount of L-lactide as specified above and preferably (also) in combination with a preferred amount of D-lactide as specified above).

The amount of acid-containing impurities in the at least one purified lactide stream(s) is low when compared to the amount of acid-containing impurities in the crude lactide stream. The acid-containing impurities may be present in the at least one purified lactide stream(s) in an amount such that each purified lactide stream has a free acid content of up to 100 meq/kg, in particular up to 50 meq/kg, more in particular up to 25 meq/kg, even more in particular up to 10 meq/kg.

To remove any remaining meso-lactide and/or acid-containing impurities from the at least one purified lactide stream(s), the purified lactide stream(s) may be subjected to one or more purification steps. Suitable purification steps include distillation and crystallization. A crystallization approach (e.g., solvent crystallization or melt-crystallization) may be preferred, as crystallization allows one to obtain a substantially pure (i.e., >99% purity) L- and/or D-lactide stream, preferably a substantially pure L-lactide stream. Typically, lactide products may be obtained with very low free acid numbers of the order of <10 meq/kg. This is advantageous, because such an L- and/or D-lactide stream can be sent to a polymerization reactor to form polylactide using little polymerization catalyst and catalyst deactivators, all of which result in polylactides with high thermal stability and little discoloration.

The meso-lactide stream comprises at least 50 wt.% of meso-lactide (based on the total weight of lactide in the stream) and preferably comprises at least 80 wt.% of meso-lactide, more preferably at least 85 wt.% of meso-lactide, even more preferably at least 90 wt.% of meso-lactide. As a maximum, the meso-lactide stream may comprise 100 wt.% of meso-lactide (based on the total weight of lactide in the stream). The meso-lactide stream may further comprise the acid-containing impurities that can be present in the crude lactide stream. These acid-containing impurities may be concentrated in the meso-lactide stream, when compared to the amount of the acid-containing impurities present in the crude lactide stream. Specifically, the amount (in wt.% based on the total weight of the meso-lactide stream) of acid-containing impurities in the meso-lactide stream may be from 1.1 to 25 times higher than the amount of acid-containing impurities in the crude lactide stream (in wt.% based on the total weight of the crude lactide stream), preferably 2 to 20 times higher, more preferably 5 to 15 times higher. The acid-containing impurities may be present in the meso-lactide stream in an amount such that the meso-lactide stream has a free acid content of from 50 to 500 meq/kg, in particular from 75 to 350 meq/kg, more in particular from 100 to 250 meq/kg.

The meso-lactide stream obtained by the separation step is subjected to an oligomerization step. The oligomerization is performed by means of a ring-opening polymerization of the meso-lactide stream. Processes suitable for such ring-opening oligomerization differ from conventional methods used to prepare lactic acid oligomers, as lactic acid oligomers are typically prepared by polycondensation of lactic acid. Ring-opening oligomerization as used in the process according to the invention is particularly useful, because water is not formed as a by-product of the oligomerization reaction. As a result thereof, it is possible to predict and control the degree of polymerization of the oligomer formed with a surprising accuracy. This, in turn, allows one to consistently obtain lactic acid oligomers having a low degree of polymerization and so optimize the total time required to convert meso-lactide into L- and D-lactide. Moreover, because no water has to be removed from the reactor in order to drive the oligomerization reaction forward, less energy is required for the oligomerization than would be required by conventional methods that rely on the polycondensation of lactic acid and do produce water as a by-product.

The meso-lactide stream obtained by the separation step may be sent directly or indirectly (preferably directly) to the oligomerization step. The meso-lactide stream may be purified or not purified (preferably not purified) before being subjected to the oligomerization step.

The meso-lactide stream obtained by the separation step may or may not be combined with further lactide streams in the oligomerization step. Further lactide streams are streams that contain at least 80 wt.% of lactide, in particular at least 90 wt.%. It may be preferred for any further lactide streams to comprise at least 50 wt.% of meso-lactide, preferably at least 80 wt.%, more preferably at least 90 wt.%.

It is preferred that the feed to the oligomerization step consists for at least 60 wt.% of meso-lactide stream directly or indirectly obtained from the separation step and for at most 40 wt.% of further lactide streams. For reasons of processing efficiency, it may be preferred for the wt.% of further lactide streams to be at most 20 wt.% of the feed to the oligomerization step, in particular at most 10 wt.%, more in particular at most 5 wt.%, even more preferably at most 2%, still more preferably at most 1%. In one embodiment, no further lactide streams are added to the oligomerization step and, in particular, the feed to the oligomerization step consists of meso-lactide stream obtained from the separation step.

In one embodiment, all streams provided to the oligomerization step comprise at least 50 wt.% of meso-lactide, preferably at least 60 wt.%, more preferably at least 70 wt.%, still more preferably at least 80 wt.%, still more preferably at least 90 wt.%.

The stream oligomerized in the oligomerization step comprises at least 50 wt.% of meso-lactide (based on the total weight of lactide in the stream) and preferably comprises at least 80 wt.% of meso-lactide, more preferably at least 85 wt.% of meso-lactide, even more preferably at least 90 wt.% of meso-lactide. As a maximum, the meso-lactide stream may comprise 100 wt.% of meso-lactide (based on the total weight of lactide in the stream).

It is noted that the feed as it is provided to the oligomerization step generally has a relatively low lactic acid content. The feed as it is provided to the oligomerization step generally has a lactic acid content of at most 10 wt.%, in particular at most 5 wt.%, more in particular at most 2 wt.%, often much lower. Some lactic acid may be added to improve processing properties and flow, but this is not required, and amounts may be minimal. This is a substantial difference with the feed as it is provided to the oligomerization step in the process of WO2010/105143.

In some embodiments, the oligomerization of the meso-lactide stream takes place in a dedicated oligomerization reactor. As used herein, a "dedicated oligomerization reactor" is an oligomerization reactor to which a feed is provided, which consists for at least 50 wt.% of meso-lactide. In particular, the feed provided to the dedicated oligomerization reactor may consist for at least 80 wt.%, more in particular at least 90 wt.%, of meso-lactide.

An initiator may be used to control the chain length of the lactic acid oligomer and may be chosen from initiators known in the art. Suitable initiators may be selected from primary monoalcohols (e.g., primary C₃₋₂₀ alkyl alcohols, like 1-hexanol, 1-decanol, 2-ethyl-1-hexanol and 1-pentadecanol), polyfunctional alcohols (e.g., C₃₋₂₀ alkyl di- and/or triols, like 1,4-butanediol and 1,6-hexanediol), and hydroxy acids (e.g., C₃₋₂₀ hydroxy acids, like glycolic acid, mandelic acid and lactic acid). Lactic acid is particularly preferred for the invention as this allows a 100% lactic acid-based oligomer. Dependent on the allowable reaction time in the oligomerization reactor, catalysts may be used to increase the ring-opening oligomerization rates. Suitable catalysts are well-established in the art and will be discussed below.

The oligomerization can be conducted in a continuous, semi-continuous, or batch-wise manner. Continuous stirred tank reactors, tube reactors, and pipe reactors are suitable reactor types. The reactors may be used in series. Due to the small size of flows relative to the rest of the lactide process and due to low viscosities, the oligomerization preferably takes place in a stirred-tank reactor or in a tank reactor equipped with bottom recirculation pumps.

The oligomerization is preferably performed in a batch-wise manner, as this allows one to depolymerize the lactic acid oligomers in the same reactor and thus minimize operating costs. To facilitate a batch-wise operation of the reactor, the meso-lactide stream obtained by separating the crude lactide may be sent directly or indirectly (preferably directly) to a holding tank, where meso-lactide is collected and stored, generally under an inert atmosphere (e.g., under N₂ atmosphere or Ar atmosphere) for a predetermined amount of time (e.g., 30 minutes to 12 hours). Storage in the holding tank should be under such conditions that the meso-lactide does not react or degrade to a substantial extent. Depending on the holding time, it may be preferred to store the meso-lactide at a temperature of at most 80 °C in particular at most 70 °C, more in particular at most 60 °C. It is preferred for the lactide to be stored in liquid form. Therefore, the temperature during storage is above the melting point of meso-lactide, e.g., above 50 °C. Once a desired amount of meso-lactide has been collected in the holding tank, the collected meso-lactide can be oligomerized (in the dedicated oligomerization reactor). By operating the process according to the invention this way, a better economy of scale is achieved. By selecting the storage conditions as described above, oligomerization is prevented and increases in free acid levels (due to the formation of acids such as acetic acid and pyruvic acid) are avoided.

The oligomerization reaction is preferably carried out at a temperature in the range of 50 to 220 °C, more preferably 50 to 180 °C, even more preferably 80 to 150 °C. The pressure preferably is in the range of 1-10 bar, in particular 1-5 bar, more in particular 1-2 bar. Operation at subsatmospheric pressure is not required. The residence time of the mixture of meso-lactide and lactic acid oligomers in the oligomerization reactor is preferably 100 to 130 °C. The residence time is selected such that the oligomers formed have a desired molecular weight. The lactic acid oligomers formed may have a degree of polymerization of 2 to 80, preferably from 3 to 50, more preferably from 4 to 30, even more preferably from 5 to 20. These low degrees of polymerization are preferred, because lactic acid oligomers having a low degree of polymerization can more easily be depolymerized due to their inherently high concentration of hydroxyl end groups. This, in turn, allows one to reduce the residence time of the oligomer-containing stream in the depolymerization reactor and so make more efficient use of the reactor used for depolymerization. The degree of polymerization is calculated from the concentration of end groups as determined by titration, as is known by the skilled person. As an example, when an initiator comprising a free acid end group (e.g., lactic acid) is used as an initiator, the amount of end groups in molar equivalents per kilogram equals the average amount of moles of oligomer chains per kilogram. The inverse of this value reports the average molecular weight, which divided by 72 g/mol gives the degree of polymerization. The free acid values typically are achieved by titration using sodium methylate or potassium methylate in water-free methanol.

The oligomerization of meso-lactide is usually conducted in the presence of a catalyst. Suitable catalysts include tin(II)chloride, tin(II)bromide, tin(IV)chloride, tin(IV)bromide, tin(II)oxide, tin(II) bis(2-ethylhexanoate), butyltin tris(2-ethyl hexanoate), monobutyltin oxide, dibutyltin dilaurate, tetraphenyltin, lead(II) oxide, zinc stearate, antimony triacetate, antimony (2-ethyl hexanoate), bismuth (2-ethylhexanoate), calcium stearate, and magnesium stearate. Metal-containing catalysts as mentioned above may be used in an amount of 20 to 2000 ppm (calculated on the weight of reaction mixture). A solvent may be used for the oligomerization of meso-lactide. Suitable solvents are benzene, toluene, xylenes, and tetrahydrofuran.

The oligomer-containing stream formed in the oligomerization step may comprise at least 80 wt.% of lactic acid oligomers (based on the total weight of the oligomers and the lactides in the stream), preferably at least 85 wt.%, preferably at least 90 wt.%, more preferably at least 95 wt.%. The oligomer-containing stream may still contain residual amounts of meso-lactide. However, the oligomer-containing stream preferably comprises at most 15 wt.% of meso-lactide, more preferably at most 10 wt.% of meso-lactide, even more preferably at most 5 wt.% of meso-lactide.

The lactic acid oligomers formed in the oligomerization step may (still) be contaminated with acid-containing impurities. In some embodiments, these acid-containing impurities are removed from the lactic acid oligomers, before the lactic oligomers are depolymerized to form the product stream. This is because certain acid-containing impurities can easily be removed from the oligomers, but only with difficulty from meso-lactide. Accordingly, also disclosed herein is preferred process for processing a crude lactide stream, wherein the process comprises the steps of:
separating a crude lactide stream comprising L-lactide, D-lactide, meso-lactide, and acid-containing impurities in one or more steps to form a meso-lactide stream comprising at least 50 wt.% of meso-lactide (based on the total weight of lactide in the stream) and acid-containing impurities, and at least one purified lactide stream comprising L-lactide and D-lactide;
oligomerizing the meso-lactide stream to form an oligomer-containing stream comprising lactic acid oligomers and acid-containing impurities;
removing acid-containing impurities from the oligomer-containing stream to form a purified oligomer-containing stream; and
depolymerizing the purified oligomer-containing stream to form a product stream comprising meso-lactide, L-lactide, and D-lactide.

The oligomerization step results in the formation of an oligomer composition as intermediate. The oligomer composition is also indicated herein as the oligomer-containing stream formed in the oligomerization step.

In one embodiment, the oligomer composition has:
- a degree of polymerization of 2 to 80, preferably from 3 to 50, more preferably from 4 to 30, even more preferably from 5 to 20,
- an overall ratio between (S)-lactic acid units and ^{®}-lactic acid units in the range of 0.25:1 to 4:1, in particular 0.3:1 to 3:1, more in particular 0.5:1 to 2:1, still more in particular 0.7:1 to 1.4:1, even more in particular 0.8:1 to1.25:1, even more in particular 0.9:1 to 1.1:1, or even 0.95:1 to 1.05:1.

The oligomer composition preferably comprises at most 15 wt.% of residual meso-lactide monomer, more preferably at most 10 wt.%, even more preferably at most 5 wt.%. In one embodiment, the oligomer composition preferably comprises at most 15 wt.% of lactide monomer (total of meso, L, and D), more preferably at most 10 wt.% of lactide monomer, even more preferably at most 5 wt.% of lactide monomer. It is further preferred for the oligomer composition to comprise at least 80 wt.% of lactic acid oligomers (based on the total weight of the oligomers and the lactides in the stream), preferably at least 85 wt.%, preferably at least 90 wt.%, more preferably at least 95 wt.%. The preferences indicated above for the oligomer-containing stream formed in the oligomerization step, whether or not after optional purification steps, also apply here.

In one embodiment, the oligomer composition contains a non-zero amount of alternating meso-lactide linkages as may be quantified using methine decoupled proton NMR measurements in CDCI3. In one embodiment, the fraction of the integral of sss, iss and ssi tetrads as a function of the integral of the entire methine integral is at least 0.05. The fraction of the integral of iss/ssi tetrads as a function of the integral of the entire methine integral may, e.g., be at least 0.1, or at least 0.2. In one embodiment, the fraction of the integral of iss/ssi tetrads as a function of the integral of the entire methine integral is at most 0.9.

The lactic acid oligomers in the (optionally purified) oligomer-containing stream are depolymerized to form a product stream comprising meso-lactide, L-lactide, and D-lactide. Methods for depolymerizing lactic acid oligomers are known in the art. These methods may involve depolymerizing the lactic acid oligomer in the presence of a depolymerization catalyst. The depolymerization catalyst may be a metal catalyst also used for the oligomerization reaction (i.e., a metal-containing catalyst as defined above). Tin(II) bis(2-ethylhexanoate) is often used commercially and so is a preferred catalyst for the depolymerization reaction. The depolymerization reaction is preferably carried out at a temperature of 160 to 260 °C at a pressure of 0.5 to 10.0 kPa (5 to 100 mbar), and a residence time of 10 minutes to 8 hours. The depolymerization is preferably performed in the same reactor that was used to synthesize the lactic acid oligomer, particularly when the oligomerization and depolymerization steps are performed batch-wise. Batch-wise operation of the process significantly reduces the investment required to install equipment and so is preferred.

In the depolymerization step, the amounts of L-lactide, D-lactide, and meso-lactide formed show some dependence on temperature. While the ratio of L- and D-lactide will remain constant, the amount of meso-lactide may be optimized (minimized) by lowering the temperature of synthesis. Therefore, the depolymerization reactor is preferably carried out at a temperature of 160 to 260 °C, more preferably at a temperature of 160 to 240 °C, even more preferably at a temperature of 160 to 220 °C, still more preferably at a temperature of 160 to 200 °C.

In one embodiment, to increase the amount of L-lactide and D-lactide synthesized during the depolymerization reaction, a racemizing agent may be added, should this be desired. Suitable racemizing agents include hydroxide salts (e.g., LiOH, NaOH, KOH, Mg(OH₂), and the like, preferably NaOH) and acetate salts (e.g., sodium acetate, potassium acetate). Other suitable racemizing agents are known in the art and include metal salts of alkyl alcohols (i.e., salts of the structure X-O-R, wherein X is a metal selected from the group consisting of Li, Na, and K and wherein R is a substituted or unsubstituted C₁₋₈ alkyl, e.g., a tert-butoxide salt), pyridines (preferably 1,4-lutidine, 2,6-lutidine, 3,5-lutidine, 2,6-di-tert-butylpyridine, or 4-dimethylaminopyridine), and non-nucleophilic bases (e.g., quinuclidine, 1,4-diazabicyclo[2.2.2]octane (DABCO), 1,8-diazabicyclo[5.4.0]undec-7-ene (DBU), 1,5-diazabicyclo(4.3.0)non-5-ene (DBN), and the like). The racemizing agent may be added in an amount of from 500 to 5000 ppm, preferably 750 to 2500 ppm, more preferably 900 to 1100 ppm.

The product stream comprises meso-lactide, L-lactide, and D-lactide. The amount of meso-lactide in the product stream may be from 10 to 60 wt.% (based on the total weight of the lactides in the stream), in particular 15 to 50 wt.%, more in particular 25 to 40 wt.%. Accordingly, the product stream may comprise 40 to 90 wt.% of L-lactide and D-lactide (combined weight, based on the total weight of the lactides in the stream), in particular 50 to 85 wt.%, more in particular 60 to 75 wt.%. It will be recognized that the product stream generally comprises equal amounts of L- and D-lactide, so the above-specified weight percentages may also be referred to as the weight percentages of rac-lactide in the product stream. The amount of other lactides (i.e., L-lactide and D-lactide) over the amount of meso-lactide can be determined by means of HPLC and may be in the ratio of 1 to 6, preferably in the ratio of 1.1 to 3, more preferably in the ratio of 1.5 to 3. A high ratio is desired, as a high ratio is indicative of an efficient conversion of meso-lactide to L- and D-lactide. A low amount of oligomers in the product stream is desirable, as this simplifies downstream processing of the product stream (e.g., the separation of the meso-lactide from the L- and D-lactide). Therefore, the amount of lactic acid oligomers in the product stream is preferably less than 5 wt.%, more preferably less than 3 wt.%, even more preferably less than 1 wt.% (calculated on the total weight of the product stream). The product stream may have a free acid content of from 50 to 500 meq/kg, in particular from 75 to 350 meq/kg, more in particular from 100 to 250 meq/kg.

The product stream comprising meso-lactide, L-lactide, and D-lactide may be separated in one or more steps to form a stream comprising a racemic mixture of L-lactide and D-lactide and a second stream comprising meso-lactide. It is preferred that the product stream is separated by means of distillation or crystallization (e.g. solvent crystallization or melt crystallization). Distillation may be preferred, particularly if the amount of racemic lactide is too low to allow an economical crystallization operation. Those skilled in the art will recognize that the product stream may either be separated in a dedicated distillation process, or returned to a distillation upstream, for example a distillation section used to separate crude lactide into a meso-lactide containing stream and an L- and D-lactide stream. Crystallization may be preferred in embodiments where the product stream contains too many color-forming species and distillation would become too inefficient. Crystallization may also be preferred when the flow of the product stream is sufficiently small that long batch times in crystallization are acceptable, for example in a static melt crystallization process.

Accordingly, also disclosed herein is preferred process for processing a crude lactide stream, wherein the process comprises the steps of:
separating a crude lactide stream comprising L-lactide, D-lactide, and meso-lactide (and optionally acid-containing impurities) in one or more steps to form a meso-lactide stream at least 50 wt.% of meso-lactide (based on the total weight of lactide in the stream) (and optionally acid-containing impurities) and at least one purified lactide stream comprising L-lactide and/or D-lactide;
oligomerizing the meso-lactide stream to form an oligomer-containing stream comprising lactic acid oligomers (and optionally acid-containing impurities);
optionally removing acid-containing impurities from the oligomer-containing stream to form a purified oligomer-containing stream;
depolymerizing the (optionally purified) oligomer-containing stream to form a product stream comprising meso-lactide, L-lactide, and D-lactide; and
separating the product stream in one or more steps to form a stream comprising a racemic mixture of L-lactide and D-lactide, and a second stream comprising meso-lactide.

Additionally or alternatively, it may be desired to increase the content of D-lactide in an L-lactide and D-lactide stream coming from a distillation section, e.g., a purified L-lactide and/or D-lactide stream as defined herein. This may be achieved by recycling at least a portion of the product stream to such distillation section, its amount dependent on the target ratio of D-lactide, L-lactide, and meso-lactide in the stream exiting the distillation section, e.g., the purified L-lactide and/or D-lactide stream.

The stream comprising a racemic mixture of L-lactide and D-lactide may be a stream wherein L- and D-lactide make up at least 80 wt.% of the stream, preferably at least 85 wt.%, more preferably at least 90 wt.%, even more preferably at least 95 wt.%. In preferred embodiments, the stream comprising stream a racemic mixture of L-lactide and D-lactide comprises 5 wt.% or less of meso-lactide (based on the total weight of lactide in the stream), preferably 4 wt.% or less of meso-lactide, more preferably 3 wt.% or less of meso-lactide, even more preferably 2 wt.% or less of meso-lactide. The acid-containing impurities may be present in the stream comprising a racemic mixture of L-lactide and D-lactide in an amount such that the stream has a free acid content of from 1 to 50 meq/kg, in particular from 1 to 25 meq/kg, more in particular from 1 to 10 meq/kg, even more in particular 1 to 10 meq/kg.

Any remaining meso-lactide and/or acid-containing impurities in the stream comprising a racemic mixture of L-lactide and D-lactide may be removed by means of one or more purification steps. Suitable purification methods include distillation and crystallization. A crystallization approach (e.g., solvent crystallization or melt-crystallization) may be preferred, as crystallization allows one to obtain a substantially pure (i.e., >99% purity) racemic L- and D-lactide stream. This is advantageous, because such a pure, racemic L- and D-stream can be sent to a polymerization reactor to form an almost colorless polylactide. A combination of distillation and crystallization may also be desired, e.g. where distillation is used to remove at least a fraction of the remaining meso-lactide and as such purify the feed to allow crystallization of rac-lactide (i.e., the racemic mixture of L-lactide and D-lactide).

The second stream comprising meso-lactide may comprise at least 50 wt.% of meso-lactide (based on the total weight of lactide in the stream), preferably at least 80 wt.%, more preferably at least 85 wt.% of meso-lactide, more preferably at least 90 wt.% of meso-lactide, even more preferably at least 95 wt.% of meso-lactide. In preferred embodiments, the acid-containing impurities are concentrated in the second stream comprising meso-lactide, such that the second stream has a free acid content of from 10 to 500 meq/kg, in particular from 20 to 250 meq/kg, more in particular from 50 to 100 meq/kg. The total amount of acid-containing impurities in the second stream comprising meso-lactide may be less than 4 wt.% (based on the total weight of the second stream). Examples of impurities that generally end up in the second stream are (monomeric) lactic acid, acetic acid, and succinic acid.

At least a portion of the second stream comprising meso-lactide may be recycled, directly or indirectly, to an earlier stage of the process. For example, at least a portion of the second stream may be recycled to a separation unit for separating the crude lactide, a holding tank (wherein meso-lactide may be stored until it is oligomerized), and/or the oligomerization step. If the process is operated in a batch-wise manner, it may be preferred to recycle the second stream to the holding tank. Recycling of the second stream may, in particular, be preferred if the oligomer-containing stream contains any acid-containing impurities and these impurities are not removed. Additionally or alternatively, at least a portion of the second stream comprising meso-lactide may be purged.

Any remaining L- and/or D-lactide, as well as any remaining acid-containing impurities, in the second stream may be removed by means of one or more purification steps. Any remaining L-lactide and/or D-lactide could be removed via distillation or crystallization. Any remaining acid-containing impurities may, for example, be removed by solvent- or melt-crystallization, as demonstrated in EP 21195962.2. The resulting purified meso-lactide stream may be subjected to polymerization, alone or in combination with a purified L-lactide and/or D-lactide stream and/or a stream comprising a racemic mixture of L-lactide and D-lactide as defined herein. If any acid-containing impurities are removed from the oligomer-containing stream or the second stream comprising meso-lactide, at least a portion of the second stream comprising meso-lactide may be sent to a polymerization reactor. In a polymerization reactor, the meso-lactide can be subjected to polymerization conditions as discussed below to form a (colorless) amorphous polylactide or combined with L- and/or D-lactide to form a more crystalline polylactide.

For completeness, it is noted that the process according to the invention may be part of a comprehensive process for manufacturing lactide. Accordingly, in some embodiments, the process according to the invention comprises the steps of:
forming lactic acid oligomers;
depolymerizing the lactic acid oligomers to form a crude lactide stream comprising L-lactide, D-lactide, and meso-lactide (and optionally acid-containing impurities);
separating the crude lactide stream in one or more steps to form a meso-lactide stream at least 50 wt.% of meso-lactide (based on the total weight of lactide in the stream) (and optionally acid-containing impurities) and at least one purified lactide stream comprising L-lactide and/or D-lactide;
oligomerizing the meso-lactide stream to form an oligomer-containing stream comprising lactic acid oligomers (and optionally acid-containing impurities);
optionally removing acid-containing impurities from the oligomer-containing stream to form a purified oligomer-containing stream;
depolymerizing the (optionally purified) oligomer-containing stream to form a product stream comprising meso-lactide, L-lactide, and D-lactide; and
separating the product stream in one or more steps to form a stream comprising a racemic mixture of L-lactide and D-lactide, and a second stream comprising meso-lactide.

The lactic acid oligomers formed in the first step of this comprehensive process may have the same features as defined above for the lactic acid oligomers of the oligomer-containing stream and are formed in an oligomerization reactor. These lactic acid oligomers are preferably formed by the polycondensation of lactic acid. The depolymerization of the lactic acid oligomers formed in this first step may be performed under the same conditions as defined above for the depolymerization of the oligomer-containing stream and generally takes place in a dedicated depolymerization reactor. Preferences for the other steps of this comprehensive process are the same as defined above.

Also disclosed herein is a lactide, preferably a racemic mixture of L-lactide and D-lactide, obtainable by the process for processing a crude lactide stream according to the invention.

### Process for producing a polylactide

The process for processing a crude lactide described above results in the formation of a number of lactide streams that are suitable for the synthesis of commercial-grade polylactides. Therefore, disclosed herein is also a process for producing a polylactide, wherein the process comprises obtaining at least one stream according to the process for processing a crude lactide according to the invention, providing at least a portion of the at least one stream, directly or indirectly, to a polymerization reactor, and forming a polylactide.

In some embodiments, the process for producing a polylactide comprises
a) obtaining at least one purified stream comprising L-lactide and/or D-lactide according to the process for processing a crude lactide according to the invention, providing at least portion of the at least one purified stream, directly or indirectly, to a polymerization reactor, and forming a polylactide; and/or
b) obtaining the stream comprising a racemic mixture of L-lactide and D-lactide according to the process for processing a crude lactide according to the invention, providing at least a portion of the stream, directly or indirectly, to a polymerization reactor, and forming a polylactide.

As discussed above, the properties of a polylactide are largely determined by the ratio of (S)-lactic acid units to (R)-lactic acid units in the polymer. Therefore, it is preferred that at least 80% (in particular, at least 90%) of the lactic acid monomers in the polylactide are (S)-lactic acid units or (R)-lactic acid units, preferably (S)-lactic acid units, the remainder of the lactic acid monomers in the polylactide being the opposite enantiomer. To obtain a polylactide with these amounts of (S)- and (R)-lactic acid units, various streams obtained according to the process for processing a crude lactide may be combined, either with other streams obtained according to the process or with stockpiled L-lactide, D-lactide and/or meso-lactide. For example, the polylactide may be synthesized from a mixture comprising 0-20 wt.%, preferably 5-20 wt.%, of meso-lactide and 80-100 wt.%, preferably 80-95 wt.%, of L- and/or D-lactide, preferably L-lactide. Uniquely, the process of the invention allows production of polylactide using only L-lactide and rac-lactide, without suffering significant yield loss through meso-lactide. This is desired as meso-lactide streams typically are known to contain the most color-forming impurities.

When the amount of meso-lactide in the product stream is low (e.g., <2 wt.%), it may be purged and polylactides may be produced out of (only) a combination of racemic lactide and L-lactide, and optionally D-lactide. Commercial PLA products then demand typically from 2 to 15% D-lactide in the final mix for polymerization. The process for producing a polylactide according to the invention may then comprise the steps of
obtaining the at least one purified stream comprising L-lactide and/or D-lactide according to the process for processing a crude lactide stream according to the invention;
obtaining the stream comprising a racemic mixture of L-lactide and D-lactide according to the process for processing a crude lactide stream according to the invention;
combining at least a portion of the at least one purified stream with L-lactide and/or D-lactide and at least a portion of the stream comprising a racemic mixture of L-lactide and D-lactide to form a polymerizable stream; and
polymerizing the polymerizable stream to form a polylactide, optionally wherein the polymerizable stream comprises 1-20 wt.% of D-lactide (based on the total amount of lactide in the stream), preferably 2 to 15 wt.% of D-lactide.

Generally, polymerization will be carried out by providing the lactides to a polymerization reactor, where they will be subjected to polymerization conditions, usually in the presence of a polymerization catalyst. Suitable polymerization conditions are known in the art. They may, for example, include reacting the lactide at a temperature of 100 to 225 °C, in particular 120 to 220 °C, more in particular 130 to 210 °C. Suitable polymerization catalysts are also known in the art. The catalysts described above for the oligomerization of meso-lactide may also be used here and are optionally used in catalytically effective amounts, e.g., 1 to 2000 ppm (calculated on the weight of the monomer). The polymerization reaction is usually allowed to continue until the governing thermal equilibrium concentration of residual lactide is reached, typically between 3 and 8 wt.% at the temperatures mentioned. Once the desired conversion is reached, the polymerization catalyst is often deactivated through the addition of a catalyst deactivating agent, as this stabilizes the polylactide product against catalyzed back-biting, allows low residual lactide levels below 0.5 wt.% and accordingly affords the final product a thermal stability guaranteeing suitability for melt processing at PLA converters. The resulting polylactide may have an absolute number average molecular weight (Mn) of from 20 to 150 kg/mol, preferably from 35 to 100 kg/mol, as determined by gel permeation chromatography using light scattering detection.

In some embodiments, the processes for processing a crude lactide and for producing a polylactide are combined to form an integrated process. Such an integrated process for producing a polylactide may comprise the steps of:
forming lactic acid oligomers;
depolymerizing the lactic acid oligomers to form a crude lactide stream comprising L-lactide, D-lactide, and meso-lactide (and optionally acid-containing impurities);
separating the crude lactide stream in one or more steps to form a meso-lactide stream at least 50 wt.% of meso-lactide (based on the total weight of lactide in the stream) (and optionally acid-containing impurities) and at least one purified lactide stream comprising L-lactide and/or D-lactide;
oligomerizing the meso-lactide stream to form an oligomer-containing stream comprising lactic acid oligomers (and optionally acid-containing impurities);
optionally removing acid-containing impurities from the oligomer-containing stream to form a purified oligomer-containing stream;
depolymerizing the (optionally purified) oligomer-containing stream to form a product stream comprising meso-lactide, L-lactide, and D-lactide;
separating the product stream in one or more steps to form a stream comprising a racemic mixture of L-lactide and D-lactide, and a second stream comprising meso-lactide;
optionally, purifying the stream comprising a racemic mixture of L-lactide and D-lactide;
combining L-lactide (e.g. a purified lactide stream comprising L-lactide as obtained by separating the crude lactide) and at least part of the (purified) stream comprising a racemic mixture of L-lactide and D-lactide to form a polymerizable stream (optionally comprising from 2.0 to 15 wt.% of D-lactide, calculated on the total weight of the polymerizable stream);
polymerizing the polymerizable stream to form a polylactide.

The lactic acid oligomers formed in the first step of this integrated process may have the same features as defined above for the lactic acid oligomers of the oligomer-containing stream and are formed in an oligomerization reactor. These lactic acid oligomers are preferably formed by the polycondensation of lactic acid. The depolymerization of the lactic acid oligomers formed in this first step may be performed under the same conditions as defined above for the depolymerization of the oligomer-containing stream and generally takes place in a dedicated depolymerization reactor. Preferences for the other steps of this integrated process are the same as defined above.

Also disclosed herein is a polylactide obtainable by the process for producing a polylactide according to the invention.

### Process for producing lactic acid

The stream comprising a racemic mixture of L-lactide and D-lactide obtained according to the invention may be substantially pure (i.e., have a free acid content of 10 meg/kg or less, as discussed above). By hydrolyzing this stream, a very pure, racemic mixture of lactic acid can be obtained, which is even suitable for food applications.

Therefore, also disclosed is a process for producing a racemic mixture of lactic acid, wherein the process comprises hydrolyzing at least a portion of the stream comprising a racemic mixture of L-lactide and D-lactide obtained according to the process for processing a crude lactide according to the invention to form a racemic mixture of (S)-lactic acid and (R)-lactic acid Conditions suitable for hydrolyzing lactides are known in the art.

In some embodiments, the racemic mixture of (S)-lactic acid and (R)-lactic acid is provided, directly or indirectly, to an oligomerization reactor or a depolymerization reactor.

Also disclosed herein is a racemic mixture of (S)-lactic acid and (R)-lactic acid obtainable by the process for producing a racemic mixture of (S)-lactic acid and (R)-lactic acid according to the invention.

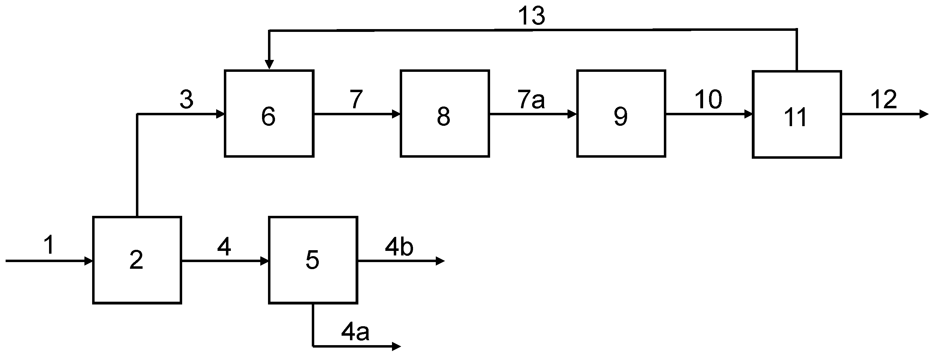

The above-disclosed processes will be illustrated with reference to the Figs. 1 to 5, without being limited thereto or thereby.

In Fig. 1, a crude lactide stream (1) comprising L-lactide, D-lactide, and meso-lactide is sent to a separation unit (2), which may e.g. be a distillation unit or a crystallization unit. The crude lactide stream (1) is separated to form a meso-lactide stream (3) comprising at least 50 wt.% of meso-lactide (based on the total weight of lactide in the stream) and at least one purified Land/or D-lactide stream (4) comprising L- and/or D-lactide, and optionally a small amount of meso-lactide (e.g., less than 2 wt.%, based on the total weight of the lactides in stream (4)). Only one purified L- and/or D-lactide stream (4) is shown. This purified L- and/or D-lactide stream (4) may be subjected to a purification step, e.g., a solvent- or melt-crystallization, in purification unit (5) to obtain a substantially pure L-lactide stream (4a) and a further stream comprising L- and/or D-lactide (4b). Both of these streams may be isolated and processed as desired. The meso-lactide stream (3) is sent to an oligomerization reactor (6), resulting in the formation of an oligomer-containing stream (7) comprising lactic acid oligomers (and optionally acid-containing impurities). This oligomer-containing stream (7) may be subjected to one or more optional purification steps. For example, the oligomer-containing stream (7) may be sent to optional purification unit (8) for the removal of any acid-containing impurities present in the oligomer-containing stream (7). The oligomer-containing stream (7) (or the purified oligomer-containing stream (7a)) is sent to a depolymerization reactor (9) (which may be the same reactor as oligomerization reactor (6)), where the lactic acid oligomers are depolymerized to form a product stream (10) comprising meso-lactide, L-lactide, and D-lactide. The product stream (10) may be sent to a further separation unit (11), e.g. a distillation unit or a crystallization unit, to separate the product stream (10) into a stream (12) comprising a racemic mixture of L-lactide and D-lactide, and a second stream (13) comprising meso-lactide. The second stream (13) comprising meso-lactide may (partly) be recycled to the oligomerization reactor (6) (as depicted in Fig. 1) and/or to the separation unit (2), and/or (partly) be subjected to a purification step, after which the resulting purified meso-lactide stream may be sent to a polymerization reactor for polymerization together with the L-lactide stream (4a) and/or the stream (12) comprising a racemic mixture of L-lactide and D-lactide.

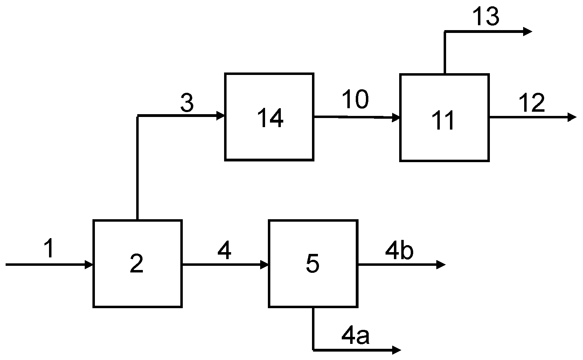

In Fig. 2, a preferred process is depicted, wherein the oligomerization of the meso-lactide stream (3) and the depolymerization of the so-obtained lactic acid oligomer occur in the same reactor. Accordingly, in Fig. 2, the meso-lactide stream (3) is provided to reactor (14), wherein the meso-lactide stream is oligomerized to form lactic acid oligomers. Then, the lactic acid oligomers are depolymerized in the same reactor (14) to form the product stream (10) comprising meso-lactide, L-lactide, and D-lactide. The product stream (10) may be sent to a further separation unit (11), e.g. a distillation unit or a crystallization unit, to separate the product stream (10) into a stream (12) comprising a racemic mixture of L-lactide and D-lactide, and a second stream (13) comprising meso-lactide, as in Fig. 1. The second stream (13) comprising meso-lactide may be recycled to reactor (14) and/or to separation unit (2), and/or (partly) be subjected to a purification step, after which the resulting purified meso-lactide stream may be sent to a polymerization reactor for polymerization together with the L-lactide stream (4a) and/or the stream (12) comprising a racemic mixture of L-lactide and D-lactide.

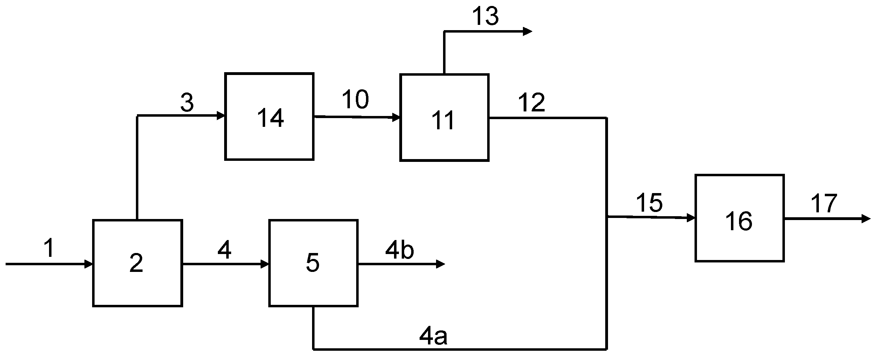

In Fig. 3, a process according to Fig. 2 is depicted, wherein the L-lactide stream (4a) and the stream (12) comprising a racemic mixture of L-lactide and D-lactide are combined to form a polymerizable stream (15) and the polymerizable stream (15) is sent to a polymerization reactor (16), where the is reacted to form a polylactide. The streams may be combined prior to entering the polymerization reactor (16) (as depicted in Fig. 3) or be combined inside the polymerization reactor (16). The polylactide product may be removed from the polymerization reactor (16) through line (17) and processed as desired.

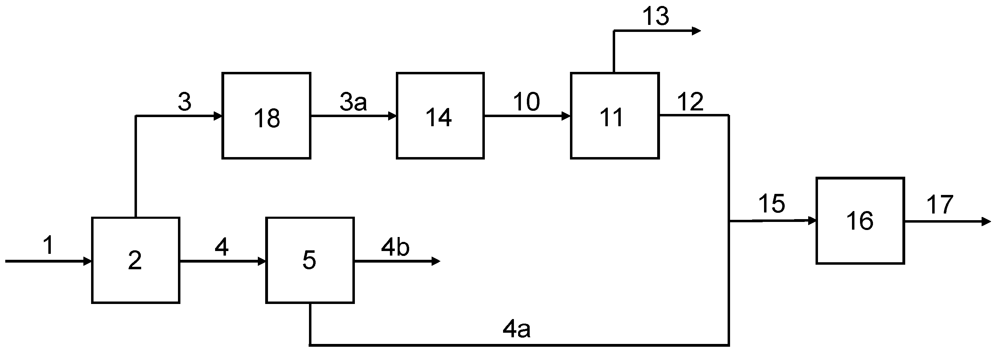

In Fig. 4, a process according to Fig. 3 depicted, wherein the process is operated in batch-wise manner and meso-lactide is collected in a holding tank prior to oligomerization. Specifically, the meso-lactide stream (3) is collected in holding tank (18), where it is stored under N₂ or Ar at a temperature from 50 to 60 °C for a predetermined amount of time. For example, the meso-lactide stream may be stored until a desired amount of meso-lactide has been collected. The collected meso-lactide stream (3a) is then sent to reactor (14), where the meso-lactide is subjected to the oligomerization-depolymerization sequence according to the invention to form product stream (10). In some embodiments, reactor (14) may be used as a holding tank (18).

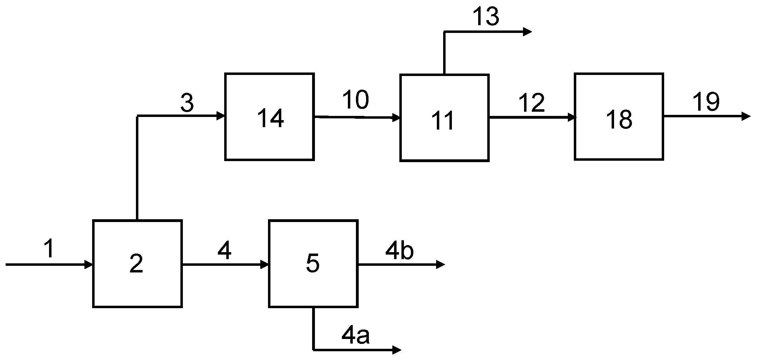

In Fig. 5, a process according to Fig. 2 is depicted, wherein at least a portion of the stream (12) comprising a racemic mixture of L-lactide and D-lactide is sent to a hydrolysis reactor (19), where the stream is hydrolyzed with water to form a racemic mixture of (S)-lactic acid and (R)-lactic acid (20), which may, for example, (partly) be sent to an oligomerization reactor (in particular, a polycondensation reactor) or to a depolymerization reactor used to prepare the crude lactide. The racemic mixture of (S)- and (R)-lactic acid (19) may be also isolated and processed as desired, e.g. in food applications.

When amounts, concentrations, dimensions and other parameters are expressed in the form of a range, a preferable range, an upper limit value, a lower limit value or preferable upper and lower limit values, it should be understood that any ranges obtainable by combining any upper limit or preferable value with any lower limit or preferable value are also specifically disclosed, irrespective of whether the obtained ranges are clearly mentioned in the context. In addition, it should be understood that all percentages mentioned herein are weight percentages, unless specified otherwise.

### Examples

The following examples will illustrate the practice of the invention in some preferred embodiments and are not intended to be limiting. Other embodiments within the scope of the invention will be apparent to the skilled person.

### Chemicals and methods

Meso-lactide used for these studies was obtained from Total Corbion PLA bv as received; it showed a free acid content of 0.85 (expressed as lactic acid) and a purity of >90% meso-lactide. Heat-stable lactic acid from Corbion (HS100) was used to control the degree of polymerization.

### Example 1: Ring-opening oligomerization

A four-necked, round-bottom flask is equipped with temperature probe, temperature control unit. overhead stirring, a Liebig condenser and a heating mantle. The condenser is cooled by tap-water to condense formed water.

To start the oligomerization, a round-bottom flask, fitted with a temperature probe and overhead stirring is filled with meso-lactide and 7.7 wt.% of heat-stable lactic acid (HS100, Corbion, as received). Subsequently 600 ppm of tin(II) bis(2-ethylhexanoate) is added and the mixture is heated to 180°C while stirring and kept at this temperature for 4 hours. The reaction was carried out at atmospheric pressure.

The reaction is stopped by removing the heat source and allowing the mixture to cool to room temperature. The degree of polymerization in the so-obtained oligomer-containing stream could be reproducibly obtained in the range of 10-12. The total amount of lactide monomers (meso-, L- and D-) in the oligomer-containing stream was typically 2.2 wt.%.

This example shows the concept of producing lactic acid oligomers from meso-lactide with a predictable degree of polymerization.

### Example 2: Lactide synthesis using (only) tin(II) bis(2-ethylhexanoate)

The same round-bottom flask that was used for the oligomerization is now equipped with a 10cm Vigreux column and Liebig condenser, which is set at 96 °C to condense lactide. The condenser, on its turn, is connected to a measuring cylinder where the lactide is collected. The top of this cylinder is connected, through a cold trap, with a vacuum pump.

To start the lactide synthesis, the oligomer-containing stream (here: the product of Example 1) is gradually heated to 210 °C and, once molten, overhead stirring is started and slowly increased to 100 rpm. At a melt temperature of 210 °C, the vacuum is lowered to 5 mbar at a rate of 100 mbar per minute. The moment when the vacuum reaches 5 mbar was denoted as t=0. To prevent the set-up from clogging by solidified lactide, infrared heating lights are placed at any cold spots.

At set time intervals, the amount (volume) of lactide in the cylinder is measured, as well as the melt and top temperatures of the experiment, and the vacuum. After 140 minutes of synthesis, the reaction is stopped by removing the heat source. Lactide in the so-obtained product stream was analyzed with respect to stereochemical purity, amount of meso-lactide and the sum of Land D-lactide. Lactide was obtained with 94% yield (based on starting amount of oligomer), its stereochemical purity was 55% L-lactate. The lactide contained 55.5 wt.% meso-lactide and 40.6 wt.% L- and D-lactide and some minor amounts of other compounds (<4 wt.% in total), mostly lactic acid and smaller oligomers.

This example shows that, using the process according to the invention, a high conversion to lactide is obtained and about 40.6 wt.% of meso-lactide could be converted to rac-lactide.

### Example 3: Crude lactide synthesis using tin octoate and sodium hydroxide to racemize

The protocol of Example 1 was now repeated but simultaneous with the addition of 600ppm tin octoate, 1000ppm sodium hydroxide is added to the four-necked round-bottom flask. After 4 hours reaction time at 180°C, the oligomerization reaction was completed and the crude lactide protocol of Example 2 was repeated. After 140min synthesis time, crude lactide was again obtained in high, albeit slightly lower, yield (75%) but with significantly higher proportion of L- and D-lactide (55.4%) and thus lower amount of meso-lactide (39%).

This example shows that the amount of rac-lactide produced may be further increased by addition of a racemization agent.

## Claims

1. A process for processing a crude lactide stream, wherein the process comprises the steps of:
separating a crude lactide stream comprising 75-95 wt.% of L-lactide (based on the total weight of lactide in the stream), 0.01 to 5 wt.% of D-lactide (based on the total weight of lactide in the stream), and 1-25 wt.% of meso-lactide (based on the total weight of lactide in the stream) in one or more steps to form a meso-lactide stream comprising at least 50 wt.% of meso-lactide (based on the total weight of lactide in the stream) and at least one purified lactide stream comprising L-lactide and/or D-lactide, wherein the amount of meso-lactide in the at least one purified L- and/or D lactide stream is at most 50% of the amount of meso-lactide in the crude lactide stream;
oligomerizing the meso-lactide stream to form an oligomer-containing stream comprising lactic acid oligomers; and
depolymerizing the oligomer-containing stream to form a product stream comprising meso-lactide, L-lactide and D-lactide.

2. The process according to claim 1, wherein the process further comprises separating the product stream in one or more steps to form a stream comprising a racemic mixture of L-lactide and D-lactide, and a second stream comprising meso-lactide.

3. The process according to claim 2, wherein the product stream is separated by means of distillation, crystallization or a combination thereof, preferably solvent-crystallization, melt-crystallization, or distillation followed by (solvent or melt) crystallization.

4. The process according to claim 2 or 3, wherein the process further comprises recycling the second stream comprising meso-lactide to the step of separating the crude lactide stream and/or to the step of oligomerizing the meso-lactide stream.

5. The process according to any one of claims 1 to 4, wherein the lactic acid oligomers have a degree of polymerization of from 2 to 80, preferably from 5 to 20, as calculated from the concentration of end-groups as determined by titration.

6. The process according to any one of claims 1 to 5, wherein the product stream comprises from 40 to 90 wt.% of a combined amount of L-lactide and D-lactide (based on the total weight of lactide in the stream).

7. The process according to any one of claims 1 to 6, wherein the stream comprising a racemic mixture of L-lactide and D-lactide has a free acid content of less than 25 meq/kg, preferably less than 10 meq/kg, as determined by titration.

8. The process according to any one of claims 1 to 7, wherein the crude lactide stream is obtained by the steps of
forming lactic acid oligomers; and
depolymerizing the lactic acid oligomers to form the crude lactide stream comprising L-lactide, D-lactide, and meso-lactide.

9. The process according to any one of claims 1 to 8, wherein the oligomerization is operated in a batch-wise manner, and wherein the process comprises, prior to oligomerization, the step of storing the meso-lactide stream in a holding tank under an inert atmosphere, preferably under N2 atmosphere, at a temperature from 50 to 80 °C for a predetermined amount of time, preferably for 30 minutes to 12 hours.

10. A process for producing a polylactide, wherein the process comprises the steps of obtaining a product stream according to the process of any one of claims 1 to 9, providing at least a portion of said stream, directly or indirectly, to a polymerization reactor, and forming a polylactide.

11. The process according to claim 10, wherein the process comprises:
a) obtaining at least one purified stream comprising L-lactide and/or D-lactide according to the process of any one of claims 1 to 9, providing at least a portion of the at least one purified stream, directly or indirectly, to a polymerization reactor, and forming a polylactide; and/or
b) obtaining the stream comprising a racemic mixture of L-lactide and D-lactide according to the process of any one of claims 2 to 9, providing at least a portion of the stream, directly or indirectly, to a polymerization reactor, and forming a polylactide.

12. The process according to claim 11, wherein the process comprises
obtaining the at least one purified stream comprising L-lactide and/or D-lactide according to the process of any one of claims 1 to 9;
obtaining the stream comprising a racemic mixture of L-lactide and D-lactide according to the process of any one of claims 2 to 9;
combining at least a portion of the at least one purified stream with L-lactide and/or D-lactide and at least a portion of the stream comprising a racemic mixture of L-lactide and D-lactide to form a polymerizable stream; and
polymerizing the polymerizable stream to form a polylactide, optionally wherein the polymerizable stream comprises 1-20 wt.% of D-lactide (based on the total amount of lactide in the stream).

13. A process for producing a racemic mixture of (S)-lactic acid and (R)-lactic acid, wherein the process comprises hydrolyzing at least a portion of the stream comprising a racemic mixture of L-lactide and D-lactide obtained according to the process of any one of claims 2 to 9 to form a racemic mixture of (S)-lactic acid and (R)-lactic acid.

14. The process according to claim 13, wherein the racemic mixture of (S)-lactic acid and (R)-lactic acid is provided, directly or indirectly, to an oligomerization reactor or a depolymerization reactor.

## Patentansprüche

1. Ein Verfahren zur Aufbereitung eines rohen Lactidstroms, wobei das Verfahren die Schritte umfasst:
Trennen eines rohen Lactid-Stroms, der 75-95 Gew.-% L-Lactid (bezogen auf das Gesamtgewicht an Lactid in dem Strom), 0,01 bis 5 Gew.-% D-Lactid (bezogen auf das Gesamtgewicht an Lactid in dem Strom) und 1-25 Gew.-% meso-Lactid (bezogen auf das Gesamtgewicht an Lactid in dem Strom) umfasst, in einem oder mehreren Schritten, um einen meso-Lactidstrom, der mindestens 50 Gew.-% Meso-Lactid (bezogen auf das Gesamtgewicht an Lactid in dem Strom) umfasst, und mindestens einen gereinigten Lactid-Strom, der L-Lactid und/oder D-Lactid umfasst, zu bilden, wobei die Menge an meso-Lactid in dem mindestens einen gereinigten L- und/oder D-Lactidstrom höchstens 50% der Menge an meso-Lactid in dem rohen Lactidstrom beträgt;
Oligomerisieren des meso-Lactidstroms, um einen oligomerhaltigen Strom zu bilden, der Milchsäureoligomere umfasst; und
Depolymerisieren des oligomerhaltigen Stroms, um einen Produktstrom zu bilden, der meso-Lactid, L-Lactid und D-Lactid umfasst.

2. Das Verfahren nach Anspruch 1, wobei das Verfahren ferner Trennen des Produktstroms in einem oder mehreren Schritten umfasst, um einen Strom, der ein racemisches Gemisch aus L-Lactid und D-Lactid umfasst, und einen zweiten Strom, der meso-Lactid umfasst, zu bilden.

3. Das Verfahren nach Anspruch 2, wobei der Produktstrom mittels Destillation, Kristallisation oder einer Kombination davon, vorzugsweise Lösungsmittelkristallisation, Schmelzkristallisation oder Destillation gefolgt von (Lösungsmittel- oder Schmelz-)Kristallisation, getrennt wird.

4. Das Verfahren nach Anspruch 2 oder 3, wobei das Verfahren ferner Rückführen des zweiten Stroms, der meso-Lactid umfasst, in den Schritt des Trennens des rohen Lactidstroms und/oder in den Schritt des Oligomerisierens des meso-Lactidstroms umfasst.

5. Das Verfahren nach einem der Ansprüche 1 bis 4, wobei die Milchsäureoligomere einen Polymerisationsgrad von 2 bis 80, vorzugsweise von 5 bis 20, aufweisen, wie aus der Konzentration an Endgruppen, wie durch Titration bestimmt, berechnet.

6. Das Verfahren nach einem der Ansprüche 1 bis 5, wobei der Produktstrom 40 bis 90 Gew.-% einer kombinierten Menge an L-Lactid und D-Lactid (bezogen auf das Gesamtgewicht an Lactid in dem Strom) umfasst.

7. Das Verfahren nach einem der Ansprüche 1 bis 6, wobei der Strom, der ein racemisches Gemisch aus L-Lactid und D-Lactid umfasst, einen Gehalt an freier Säure von weniger als 25 mÄq/kg, vorzugsweise weniger als 10 mÄq/kg, aufweist, wie durch Titration bestimmt.

8. Das Verfahren nach einem der Ansprüche 1 bis 7, wobei der rohe Lactidstrom durch die Schritte
Bilden von Milchsäureoligomeren und
Depolymerisieren der Milchsäureoligomere, um den rohen Lactidstrom, der L-Lactid, D-Lactid und meso-Lactid umfasst, zu bilden,
erhalten wird.

9. Das Verfahren nach einem der Ansprüche 1 bis 8, wobei die Oligomerisation chargenweise durchgeführt wird und wobei das Verfahren vor der Oligomerisation den Schritt des Lagerns des meso-Lactidstroms in einem Vorratsbehälter unter einer inerten Atmosphäre, vorzugsweise unter N2-Atmosphäre, bei einer Temperatur von 50 bis 80°C für eine vorbestimmte Zeitdauer, vorzugsweise für 30 Minuten bis 12 Stunden, umfasst.

10. Ein Verfahren zur Herstellung eines Polylactids, wobei das Verfahren die Schritte des Erhaltens eines Produktstroms gemäß dem Verfahren nach einem der Ansprüche 1 bis 9, des direkten oder indirekten Zuführens mindestens eines Teils des Stroms zu einem Polymerisationsreaktor und des Bildens eines Polylactids umfasst.

11. Das Verfahren nach Anspruch 10, wobei das Verfahren umfasst:
a) Erhalten mindestens eines gereinigten Stroms, der L-Lactid und/oder D-Lactid umfasst, gemäß dem Verfahren nach einem der Ansprüche 1 bis 9, direktes oder indirektes Zuführen mindestens eines Teils des mindestens einen gereinigten Stroms zu einem Polymerisationsreaktor und Bilden eines Polylactids; und/oder
b) Erhalten des Stroms, der ein racemisches Gemisch aus L-Lactid und D-Lactid umfasst, gemäß dem Verfahren nach einem der Ansprüche 2 bis 9, direktes oder indirektes Zuführen mindestens eines Teils des Stroms zu einem Polymerisationsreaktor und Bilden eines Polylactids.

12. Das Verfahren nach Anspruch 11, wobei das Verfahren umfasst
Erhalten des mindestens einen gereinigten Stroms, der L-Lactid und/oder D-Lactid umfasst, gemäß dem Verfahren nach einem der Ansprüche 1 bis 9;
Erhalten des Stroms, der ein racemisches Gemisch aus L-Lactid und D-Lactid umfasst, gemäß dem Verfahren nach einem der Ansprüche 2 bis 9;
Zusammenführen mindestens eines Teils des mindestens einen gereinigten Stroms mit L-Lactid und/oder D-Lactid und mindestens eines Teils des Stroms, der ein racemisches Gemisch aus L-Lactid und D-Lactid umfasst, um einen polymerisierbaren Strom zu bilden; und
Polymerisieren des polymerisierbaren Stroms, um ein Polylactid zu bilden, wobei der polymerisierbare Strom gegebenenfalls 1-20 Gew.-% D-Lactid (bezogen auf die Gesamtmenge an Lactid in dem Strom) umfasst.

13. Ein Verfahren zur Herstellung eines racemischen Gemischs aus (S)-Milchsäure und (R)-Milchsäure, wobei das Verfahren Hydrolysieren mindestens eines Teils des Stroms, der ein racemisches Gemisch aus L-Lactid und D-Lactid umfasst, das gemäß dem Verfahren nach einem der Ansprüche 2 bis 9 erhalten wurde, um ein racemisches Gemisch aus (S)-Milchsäure und (R)-Milchsäure zu bilden, umfasst.

14. Das Verfahren nach Anspruch 13, wobei das racemische Gemisch aus (S)-Milchsäure und (R)-Milchsäure direkt oder indirekt einem Oligomerisationsreaktor oder einem Depolymerisationsreaktor zugeführt wird.

## Revendications

1. Procédé de traitement d'un flux de lactide brut, dans lequel le procédé comprend les étapes suivantes :
la séparation d'un flux de lactide brut comprenant 75 à 95 % en poids de L-lactide (sur la base du poids total de lactide dans le flux), 0,01 à 5 % en poids de D-lactide (sur la base du poids total de lactide dans le flux) et 1 à 25 % en poids de méso-lactide (sur la base du poids total de lactide dans le flux) en une ou plusieurs étapes pour former un flux de méso-lactide comprenant au moins 50 % en poids de méso-lactide (sur la base du poids total de lactide dans le flux) et au moins un flux de lactide purifié comprenant du L-lactide et/ou du D-lactide, dans lequel la quantité de méso-lactide dans l'au moins un flux de L- et/ou de D-lactide purifié est d'au plus 50 % de la quantité de méso-lactide dans le flux de lactide brut ;
l'oligomérisation du flux de méso-lactide pour former un flux contenant des oligomères comprenant des oligomères d'acide lactique ; et
la dépolymérisation du flux contenant des oligomères pour former un flux de produit comprenant du méso-lactide, du L-lactide et du D-lactide.

2. Procédé selon la revendication 1, dans lequel le procédé comprend en outre la séparation du flux de produit en une ou plusieurs étapes pour former un flux comprenant un mélange racémique de L-lactide et de D-lactide, et un second flux comprenant du méso-lactide.

3. Procédé selon la revendication 2, dans lequel le flux de produit est séparé au moyen d'une distillation, d'une cristallisation ou d'une combinaison de celles-ci, de préférence une cristallisation par solvant, une cristallisation par fusion ou une distillation suivie d'une cristallisation (par solvant ou par fusion).

4. Procédé selon la revendication 2 ou 3, dans lequel le procédé comprend en outre le recyclage du second flux comprenant du méso-lactide jusqu'à l'étape de séparation du flux de lactide brut et/ou jusqu'à l'étape d'oligomérisation du flux de méso-lactide.

5. Procédé selon l'une quelconque des revendications 1 à 4, dans lequel les oligomères d'acide lactique ont un degré de polymérisation de 2 à 80, de préférence de 5 à 20, tel que calculé à partir de la concentration de groupes terminaux telle que déterminée par titrage.

6. Procédé selon l'une quelconque des revendications 1 à 5, dans lequel le flux de produit comprend de 40 à 90 % en poids d'une quantité combinée de L-lactide et de D-lactide (sur la base du poids total de lactide dans le flux).

7. Procédé selon l'une quelconque des revendications 1 à 6, dans lequel le flux comprenant un mélange racémique de L-lactide et de D-lactide présente une teneur en acide libre inférieure à 25 meq/kg, de préférence inférieure à 10 meq/kg, telle que déterminée par titrage.

8. Procédé selon l'une quelconque des revendications 1 à 7, dans lequel le flux de lactide brut est obtenu par les étapes de
formation d'oligomères d'acide lactique ; et
dépolymérisation des oligomères d'acide lactique pour former le flux de lactide brut comprenant du L-lactide, du D-lactide et du méso-lactide.

9. Procédé selon l'une quelconque des revendications 1 à 8, dans lequel l'oligomérisation est réalisée par lots, et dans lequel le procédé comprend, avant l'oligomérisation, l'étape de stockage du flux méso-lactique dans un réservoir de maintien sous atmosphère inerte, de préférence sous atmosphère de N2, à une température de 50 à 80 °C pendant une durée prédéterminée, de préférence de 30 minutes à 12 heures.

10. Procédé de production d'un polylactide, dans lequel le procédé comprend les étapes d'obtention d'un flux de produit conformément au procédé selon l'une quelconque des revendications 1 à 9, de fourniture d'au moins une partie dudit flux, directement ou indirectement, à un réacteur de polymérisation, et de formation d'un polylactide.

11. Procédé selon la revendication 10, dans lequel le procédé comprend :
a) l'obtention d'au moins un flux purifié comprenant du L-lactide et/ou du D-lactide conformément au procédé selon l'une quelconque des revendications 1 à 9, la fourniture d'au moins une partie de l'au moins un flux purifié, directement ou indirectement, à un réacteur de polymérisation, et la formation d'un polylactide ; et/ou
b) l'obtention du flux comprenant un mélange racémique de L-lactide et de D-lactide conformément au procédé selon l'une quelconque des revendications 2 à 9, la fourniture d'au moins une partie du flux, directement ou indirectement, à un réacteur de polymérisation, et la formation d'un polylactide.

12. Procédé selon la revendication 11, dans lequel le procédé comprend :
l'obtention de l'au moins un flux purifié comprenant du L-lactide et/ou du D-lactide conformément au procédé selon l'une quelconque des revendications 1 à 9 ;
l'obtention du flux comprenant un mélange racémique de L-lactide et de D-lactide conformément au procédé selon l'une quelconque des revendications 2 à 9 ;
la combinaison d'au moins une partie de l'au moins un flux purifié avec du L-lactide et/ou du D-lactide et d'au moins une partie du flux comprenant un mélange racémique de L-lactide et de D-lactide pour former un flux polymérisable ; et
la polymérisation du flux polymérisable pour former un polylactide, dans lequel le flux polymérisable comprend facultativement 1 à 20 % en poids de D-lactide (sur la base de la quantité totale de lactide dans le flux).

13. Procédé de production d'un mélange racémique d'acide (S)-lactique et d'acide (R)-lactique, dans lequel le procédé comprend l'hydrolyse d'au moins une partie du flux comprenant un mélange racémique de L-lactide et de D-lactide obtenu conformément au procédé selon l'une quelconque des revendications 2 à 9 pour former un mélange racémique d'acide (S)-lactique et d'acide (R)-lactique.

14. Procédé selon la revendication 13, dans lequel le mélange racémique d'acide (S)-lactique et d'acide (R)-lactique est fourni, directement ou indirectement, à un réacteur d'oligomérisation ou un réacteur de dépolymérisation.
